# EUROPÄISCHE PATENTSCHRIFT

(11) **EP 1 593 397 B1**
(45) Veröffentlichungstag und Bekanntmachung des Hinweises auf die Patenterteilung: **29.12.2010**
(21) Anmeldenummer: 05009654.4
(22) Anmeldetag: 03.05.2005
(51) Int. Cl.: A61L 9/12, A61L 9/04

(54) **Vorrichtung zum Desodorieren von Räumen**
Apparatus for deodorizing rooms
Appareil pour désodoriser une pièce

(30) Priorität: 06.05.2004 DE 202004007271 U
(43) Veröffentlichungstag der Anmeldung: 09.11.2005
(73) Patentinhaber: Biothys GmbH, 77731 Willstätt (DE)
(72) Erfinder: Wuest, Robert, 67190 Rosheim (FR)
(74) Vertreter: Ziegler, Stefan Michael

(56) Entgegenhaltungen:
- EP-A- 0 775 441
- EP-A- 1 262 100
- EP-A- 1 334 734
- US-A- 3 990 848

## Beschreibung

Die Erfindung betrifft eine Vorrichtung zum Desodorieren von Räumen in Form einer Säule mit Öffnungen, aus denen durch zwangsgeförderte Luft Desodorantien, die an einem Trägermaterial adsorbiert sind, in den Raum ausgetrieben werden.

Eine Vorrichtung zum Desodorieren von Räumen ist aus der EP-A-1262100 bekannt.

Zum Entfernen bzw. Reduzieren von übelriechenden Substanzen in geschlossenen Räumen werden verschiedenartige Methoden angewandt. Beispielsweise können saugfähige Papiere, poröse Feststoffe oder gelartige Polymere mit aktiven Agentien, die mit den übelriechenden Substanzen reagieren oder diese maskieren, imprägniert werden. Die aktiven Agentien diffundieren langsam aus den Trägermaterialien heraus und entfalten in der näheren Umgebung ihre Wirksamkeit.

Nach WO 03/063920 A1 kann z.B. eine schwammartige Masse aus mit aktiven Agentien getränkten, vernetzten Polymeren in Gitter oder Netze eingelegt und diese in Klimaschächte oder Ventilationskästen eingesetzt werden. Durch die Zwangsbelüftung gelingt es, den gesamten Raum gleichmäßig zu desodorieren. Aber selbst wenn die aktiven Agentien über mehrere Wochen hinweg wirksam sind, müssen sie doch häufig umständlich ausgewechselt werden. Dies ist in gewerblichen Räumen, in denen eine hohe Geruchsbelastung auftritt, besonders lästig.

Der Erfindung lag daher die Aufgabe zugrunde, eine elegante Lösung für dieses Problem zu entwickeln.

Dieses Problem wird durch die erfindungsgemäße im Anspruch 1 definierte säulenförmige Vorrichtung zum Desodorieren von Räumen gelöst. Diese umfasst
- flüchtige Desodorantien, die an einem gelartigen Trägermaterial adsorbiert sind,
- ein am Boden der Säule angeordnetes Gebläse oder einen Ventilator, die einen Luftstrom erzeugen, welcher die Desodorantien desorbieren kann,
- eine Vielzahl von Öffnungen an der Säule, durch welche die desorbierten Desodorantien in den Raum austreten können.

Die mit den Desodorantien imprägnierten Trägermaterialien sind in einem oder mehreren Gittern oder Netzen im Innem der Säule eingebracht.

Je nach Wirksamkeit der Desodorantien reicht es aus, wenn man die erschöpften Trägermaterialien alle paar Wochen, in manchen Fällen sogar nur alle paar Monate auswechselt.

Als Desodorantien kommen die einschlägigen flüchtigen Duftstoffe und Parfüme in Frage, ferner aktive Agentien, die mit den übelriechenden Substanzen in der Luft reagieren können oder diese maskieren, wie z.B. Aldehyde, Ketone und Alkohole. Als geeignete Trägermaterialien, an denen die Desodorantien adsorbiert bzw. imprägniert sind, dienen vorzugsweise vernetzte, hydrophile Gruppen tragende Polymere, wie sie in WO 03/063920 A1 beschrieben sind.

Die säulenförmige, zweckmäßigerweise senkrecht stehende Vorrichtung ist vorzugsweise 20 bis 200 cm lang und hat einen Durchmesser von 5 bis 25 cm. Sie kann die Form eines runden Rohrs oder einer eckigen Säule haben.

Die imprägnierten Trägermaterialien sind erfindungsgemäß in Gitter oder Netze eingebracht, die im Innern der Säule ebenfalls senkrecht stehen. Je nach Durchmesser der Säule können eine oder mehrere Gitter oder Netze nebeneinander stehend vorgesehen sein. Die Säule weist eine Vielzahl von Öffnungen auf, aus denen die desorbierten Desodorantien an die Umgebungsluft zwangsgefördert austreten können. Die Öffnungen sind erfindungsgemäß seitlich an der Säule angebracht. Die Öffnungen können schlitzförmig, rund oder vieleckig sein, sie sind zweckmäßigerweise gleichmäßig seitlich am Umfang der Säule angebracht. Die Säule kann auch oben offen sein; bevorzugt ist eine Kombination dieser beiden Varianten. Die Desorption wird durch einen Luftstrom bewerkstelligt, der durch ein Gebläse oder einen Ventilator, die durch einen Motor angetrieben werden, erzeugt wird. Diese sind am Boden der Säule angebracht. Dort können Lufteintrittsöffnungen vorgesehen sein. Bevorzugt steht die Säule aber auf einem Podest, das unten offen ist, so dass die Umgebungsluft dort angesaugt werden und in die Säule eintreten kann.

Bei einer alternativen Ausführungsform wird das Gebläse bzw. der Ventilator so geschaltet, dass sie die Luft am oberen, offenen Ende der Säule ansaugen. Die Luft durchströmt dann die Säule von oben nach unten und desorbiert die Desodorantien, die durch die Öffnungen austreten. Diese Anordnung ist vor allem dort angebracht, wo sich am Boden Schmutz oder Staub befindet, der durch einen unten angesaugten Luftstrom mitgerissen werden könnte.

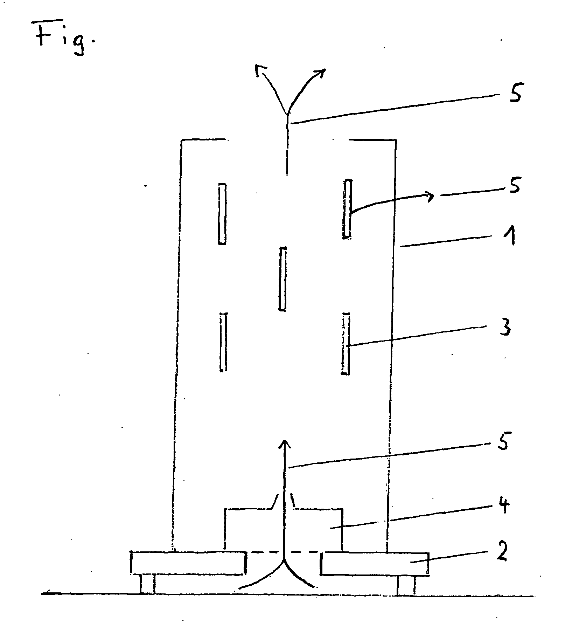

Die Figur zeigt eine Ausführungsform der erfindungsgemäßen Säule (1), welche unten und oben offen ist und auf einem Podest (2) steht. Sie weist seitlich Öffnungen (3) auf. Mit (4) ist ein Ventilator bezeichnet, der Luft durch den Boden der Säule ansaugt und einen Luftstrom (5) erzeugt. Im Innern der Säule befinden sich (nicht eingezeichnete) Gitter mit dem Trägermaterial. Der Luftstrom (5) durchströmt die Gitter von unten nach oben und desorbiert die Desodorantien, die durch Öffnungen (3) und durch die oben offene Säule austreten.
Bei der genannten alternativen Ausführungsform wird die Luft am oberen Ende der Säule angesaugt und durchströmt diese von oben nach unten.

Falls in einer bevorzugten Ausführungsform der Erfindung als Trägermaterial ein vernetztes Polymeres nach WO 03/063920 verwendet wird, liegt das imprägnierte Trägermaterial als gelartige Masse vor. Diese ist in Form von Streifen in die Gitter oder Netze eingebracht sein, oder sie wird als längliches, z.B. 1 bis 10 mm dickes Blatt eingesetzt, das spiralförmig so zusammengerollt ist, dass ein Luftstrom dazwischen durchströmen kann. Dabei kann die gelartige Masse auch auf einen Träger, z.B. auf ein Faservlies, ein Fasergewebe oder Papier aufgetragen sein.

Der Vorteil der vorliegenden Erfindung besteht nun darin, dass die gelartigen Trägermaterialien, wenn sie erschöpft sind, einfach aus der Säule herausgenommen und durch frische, mit Desodorantien beladenen Trägermaterialien ersetzt werden können. Der Zeitraum, nach dem die Trägermaterialien erschöpft sind, kann leicht durch Vorversuche bestimmt werden.

Die erfindungsgemäße Vorrichtung kann z.B. in Verkaufsräumen, Hotellobbies, Großraumbüros und Restaurants eingesetzt werden.

## Patentansprüche

1. Säulenförmige Vorrichtung zum Desodorieren von Räumen, umfassend
- flüchtige Desodorantien, die an einem gelartigen Trägermaterial adsorbiert sind,
- ein am Boden der Säule angeordnetes Gebläse oder einen Ventilator, die einen Luftstrom erzeugen, welcher die Desodorantien desorbieren kann,
- eine Vielzahl von Öffnungen, durch welche die desorbierten Desodorantien in den Raum austreten können,
**dadurch gekennzeichnet, dass** die Öffnungen Seitlich an der Säule angebracht sind, und dass die mit den Desodorantien imprägnierten Trägermaterialien in Form von Streifen oder länglichen, spiralförmig zusammengerollten Blättern in einem oder mehreren senkrecht stehenden Gittern oder Netzen im Innern der Säule eingebracht sind.

2. Säulenförmige Vorrichtung nach Anspruch 1, **dadurch gekennzeichnet, dass** die vorzugsweise senkrecht stehende Säule 20 bis 200 cm lang ist und einen Durchmesser von 5 bis 25 cm aufweist.

3. Säulenförmige Vorrichtung nach Anspruch 1, **dadurch gekennzeichnet, dass** die Öffnungen schlitzförmige, runde oder vieleckige Form haben.

4. Säulenförmige Vorrichtung nach Anspruch 1, **dadurch gekennzeichnet, dass** die Säule zusätzlich oben offen ist.

5. Säulenförmige Vorrichtung nach Anspruch 1, **dadurch gekennzeichnet, dass** die Säule auf einem Podest steht, das unten offen ist, so dass die Umgebungsluft dort angesaugt werden und in die Säule eintreten kann.

6. Säulenförmige Vorrichtung nach Anspruch 1, **dadurch gekennzeichnet, dass** die Umgebungsluft am oberen, offenen Ende der Säule angesaugt wird und in die Säule eintreten kann.

7. Säulenförmige Vorrichtung nach Anspruch 1, **dadurch gekennzeichnet, dass** die Desodorantien Duftstoffe, Parfüme oder aktive Agentien sind, die mit den in dem Raum enthaltenen übelriechenden Substanzen reagieren und diese entfernen oder maskieren können.

8. Säulenförmige Vorrichtung nach Anspruch 1, **dadurch gekennzeichnet, dass** das gelartige Trägermaterial aus einem vernetzten, hydrophile Gruppen tragenden Polymeren besteht.

## Claims

1. Column-like device for deodorising rooms, comprising
- volatile deodorants, which are adsorbed on a gel-like carrier material,
- a fan or a ventilator arranged at the base of the column, which produce an airflow which can desorb the deodorants,
- a multitude of openings, through which the desorbed deodorants can exit into the room,
**characterised in that** the openings are introduced laterally on the column, and **in that** the carrier materials impregnated with the deodorants are introduced in the form of strips or elongate sheets rolled up spirally in one or more perpendicular grids or networks in the interior of the column.

2. Column-like device according to claim 1, **characterised in that** the preferably vertical standing column is 20 to 200 cm in length and has a diameter of 5 to 25 cm.

3. Column-like device according to claim 1, **characterised in that** the openings have a slot-shaped, round or polygonal shape.

4. Column-like device according to claim 1, **characterised in that** the column is additionally open at the top.

5. Column-like device according to claim 1, **characterised in that** the column stands on a platform, which is open at the bottom, so the ambient air can be sucked in there and can enter the column.

6. Column-like device according to claim 1, **characterised in that** the ambient air is sucked in at the upper, open end of the column and can enter the column.

7. Column-like device according to claim 1, **characterised in that** the deodorants are fragrance products, perfumes or active agents, which react with the unpleasant-smelling substances contained in the room and can remove or mask them.

8. Column-like device according to claim 1, **characterised in that** the gel-like carrier material consists of a cross-linked polymer carrying hydrophilic groups.

## Revendications

1. Dispositif en forme de colonne destiné à désodoriser des pièces, comprenant :
- des désodorisants volatiles qui sont adsorbés sur un matériau de support de type gel,
- une soufflerie ou un ventilateur disposé au niveau du fond de la colonne, qui génère un flux d'air qui peut désorber les désodorisants,
- une multitude d'ouvertures par lesquelles les désodorisants désorbés peuvent sortir dans la pièce,
**caractérisé en ce que** les ouvertures sont prévues latéralement au niveau de la colonne, et **en ce que** les matériaux de support imprégnés des désodorisants sont appliqués sous la forme de bandes ou de feuilles longitudinales enroulées en spirale dans un(e) ou plusieurs grilles ou treillis verticaux à l'intérieur de la colonne.

2. Dispositif en forme de colonne selon la revendication 1, **caractérisé en ce que** la colonne de préférence en station verticale mesure 20 à 200 cm de long et présente un diamètre de 5 à 25 cm.

3. Dispositif en forme de colonne selon la revendication 1, **caractérisé en ce que** les ouvertures ont une forme de fente, une forme ronde ou polygonale.

4. Dispositif en forme de colonne selon la revendication 1, **caractérisé en ce que** la colonne est en outre ouverte sur le dessus.

5. Dispositif en forme de colonne selon la revendication 1, **caractérisé en ce que** la colonne repose sur une plate-forme qui est ouverte au niveau du dessous de façon à ce que l'air de l'environnement y soit aspiré et puisse pénétrer dans la colonne.

6. Dispositif en forme de colonne selon la revendication 1, **caractérisé en ce que** l'air de l'environnement est aspiré au niveau de l'extrémité supérieure ouverte de la colonne et peut pénétrer dans la colonne.

7. Dispositif en forme de colonne selon la revendication 1, **caractérisé en ce que** les désodorisants sont des substances odorantes, des parfums ou des agents actifs qui réagissent avec les substances d'odeur désagréable contenues dans la pièce et peuvent les éliminer ou les masquer.

8. Dispositif en forme de colonne selon la revendication 1, **caractérisé en ce que** le matériau de support de type gel se compose d'un polymère réticulé portant des groupes hydrophiles.
